# NOUVEAU FASCICULE DE BREVET EUROPEEN

(11) **EP 1 357 884 B2**
(45) Date de publication et mention de la décision concernant l'opposition: **13.11.2019**
(45) Mention de la délivrance du brevet: 28.04.2010
(21) Numéro de dépôt: 02700360.7
(22) Date de dépôt: 22.01.2002
(51) Int. Cl.: A61Q 17/04, A61K 8/46, A61K 8/68, A61K 8/81, A61K 8/891, A61Q 5/12

(54) **COMPOSITION DE TRAITEMENT DES MATIERES KERATINIQUES COMPRENANT UN POLYMERE POLY(ALKYL) VINYLLACTAME CATIONIQUE ET UN AGENT CONDITIONNEUR**
ZUSAMMENSETZUNG ZUR BEHANDLUNG VON KERATINFASERN, DIE EIN KATIONISCHES POLYVINYLLACTAM ENTHALTEN SOWIE EIN KONDITIONIERMITTEL
COMPOSITION FOR TREATING KERATINOUS MATERIALS COMPRISING A CATIONIC POLY(ALKYL) VINYLLACTAM POLYMER AND A CONDITIONING AGENT

(30) Priorité: 26.01.2001 FR 0101108
(43) Date de publication de la demande: 05.11.2003
(73) Titulaire: L'Oréal, 75008 Paris (FR)
(72) Inventeur: COTTARD, François, F-92300 Levallois-Perret (FR); DE LA METTRIE, Roland, F-78110 Le Vésinet (FR)
(74) Mandataire: L'Oreal
(86) Numéro de dépôt international: PCT/FR2002/000251
(87) Numéro de publication internationale: WO 2002/058646

(56) Documents cités:
- EP-A- 0 680 743
- WO-A-00/68282
- WO-A1-95/24882
- US-A- 4 536 390
- US-A- 4 938 953
- US-A- 4 956 430

## Description

L'invention concerne une composition de traitement des matières kératiniques, en particulier des fibres kératiniques humaines telles que les cheveux, comprenant, dans un milieu physiologiquement et en particulier cosmétiquement acceptable, au moins un agent conditionneur tel que défini dans la revendication 1, et en outre au moins un polymère poly(alkyl)vinyllactame cationique.

Ces associations permettent d'améliorer le dépôt de l'agent protecteur ou conditionneur des matières kératiniques ainsi que les propriétés cosmétiques.

Il est bien connu que les cheveux sont sensibilisés ou fragilisés à des degrés divers par l'action des agents atmosphériques et notamment de la lumière, l'eau et l'humidité ainsi que par l'action répétée des différents traitements capillaires tels que le lavage, les permanente, le défrisage, la teinture, la décoloration. De nombreuses publications divulguent que la lumière naturelle détruit certains aminoacides des cheveux. Ces agressions altérant la fibre capillaire, elles en diminuent les propriétés mécaniques comme la résistance à la traction, la charge à la rupture et l'élasticité, ou leur résistance au gonflement dans un milieu aqueux. Les cheveux sont alors ternes, rêches et cassants. Les cheveux, contrairement à la peau, éclaircissent.

On sait également que notamment la lumière et les agents de lavage ont tendance à agresser la couleur naturelle des cheveux ainsi que la couleur artificielle des cheveux teints. La couleur des cheveux s'affaiblit peu à peu ou vire vers des nuances peu esthétiques ou indésirables.

On recherche depuis de nombreuses années, dans l'industrie cosmétique des substances permettant de protéger les cheveux des dégradations engendrées par les agressions atmosphériques, telles que la lumière et la chaleur et les traitements. En particulier, on recherche des produits protégeant la couleur des fibres kératiniques colorées naturellement ou teintes artificiellement et préservant ou renforçant les propriétés mécaniques intrinsèques des fibres kératiniques (la résistance à la traction, la charge à la rupture et l'élasticité, ou leur résistance au gonflement dans un milieu aqueux).

Pour lutter contre ces dégradations de la kératine des cheveux, on a déjà proposé d'utiliser certaines substances susceptibles de filtrer les radiations lumineuses, comme l'acide 2-hydroxy-4-méthoxy benzoph'énone-5-sulfonique ou ses sels (FR-A-2 627 085) ou l'acide 4-(2-oxo-3-bornylidène méthyl) benzène sulfonique ou ses sels (EP-A-329 032) ou encore la lactoferrine (FR-A-2 673 839).

Cependant, ces filtres lorsqu'ils sont efficaces ne le sont qu'à des concentrations importantes. Or, à ces concentrations, les cheveux traités avec ces filtres présentent un toucher rêche et chargé. De plus, le démêlage est extrêmement difficile.

On a déjà préconisé dans les compositions pour le lavage ou le soin des matières kératiniques telles que les cheveux l'utilisation d'agents conditionneurs, notamment des polymères cationiques ou des silicones, pour faciliter le démêlage des cheveux et pour leur communiquer douceur et souplesse. Cependant, les avantages cosmétiques mentionnés ci-avant s'accompagnent malheureusement également, sur cheveux séchés, de certains effets cosmétiques jugés indésirables, à savoir un alourdissement de la coiffure (manque de légéreté du cheveu), un manque de tissage (cheveu non homogène de la racine à la pointe).

En outre, l'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage. Ces inconvénients sont accentués dans le cas de cheveux fins, qui manquent de nervosité et de volume. Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

US-A-4 956 430 (TAZI M.) divugue des compositions capillaires destinées au conditionnement des cheveux comprenant un terpolymère de type VP/DMAPMA/QDMAPMA-LCL* (voir en particulier les exemples 18 et 19). Ces compositions capillaires comprenent par ailleurs des agents conditionneurs tels que de la cocamidopropylbétaïne (Mitratine CB), un alcool cétylique stéarylique (Lanette Wax O) ou encore de la lauramide DEA. * VP: vinyl pyrrolidone; DMAPMA: dimethylaminopropyl methacrylamide, QDMAPMA: C9-C24 alkyl dimethylaminopropyl methacrylic acid quatemized monomers; LCL: lauryl chloride.

WO 00/68282 A (ISP INVEST INC.) traite également du conditionnement de la chevelure utilisant pour ce faire des compositions comprenant des copolymères vinyllactames cationiques associés à d'autres agents conditionneurs; parmi ces agents conditionneurs figurent l'olealkonium chloride, la lauramine oxide ou encore le GAFFIX VC-13 (voir rev. 1; exemples B,D,F).

En résumé, il s'avère que les compositions cosmétiques actuelles contenant des agents conditionneurs, ne donnent pas complètement satisfaction.

La demanderesse a maintenant découvert que l'association d'un polymère poly(alkyl)vinyllactame cationique particulier avec des agents conditionneurs permet de remédier à ces inconvénients.

Ainsi, à la suite d'importantes recherches menées sur la question, il a maintenant été trouvé par la Demanderesse qu'en introduisant un polymère particulier dans les compositions en particulier capillaires à base d'agents conditionneurs des matières kératiniques, il est possible d'augmenter le dépôt de l'agent protecteur ou conditionneur des matières kératiniques et par la même d'augmenter la protection ou le conditionnement.

Sans vouloir limiter la présente invention à une quelconque théorie, il semblerait exister lors du rinçage, des interactions et/ou des affinités particulières entre l'agent conditionneur des matières kératiniques, les polymères poly(alkyl)vinyllactames conformes à l'invention et les cheveux, qui favorisent un dépôt régulier, important et durable desdites agents protecteurs ou conditionneurs des matières kératiniques et polymères poly(alkyl)vinyllactames à la surface desdits cheveux, ce dépôt qualitatif et quantitatif étant probablement l'une des causes de l'amélioration observée au niveau des propriétés finales, en particulier la facilité de coiffage, le maintien, la nervosité et le volume des cheveux traités. Toutes ces découvertes sont à la base la présente invention.

Ainsi, selon la présente invention, il est maintenant proposé de nouvelles compositions cosmétiques, comprenant, dans un milieu physiologiquement et en particulier cosmétiquement acceptable, au moins un agent conditionneur des matières kératiniques et au moins un polymère poly(alkyl)vinyllactame cationique comprenant :
1.-a) au moins un monomère de type vinyl lactame ou alkyl(C₁-C₅)vinyllactame
2.-b) au moins un monomère choisi parmi ceux de structures (I) et (II) : 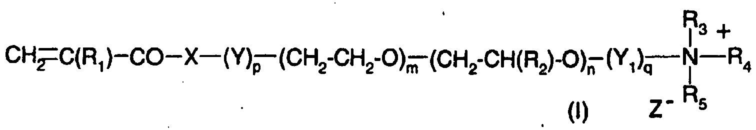 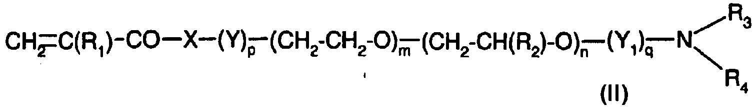 dans lesquelles :
   X désigne un atome d'oxygène ou un radical NR₆,
   R₁ et R₆ désignent indépendamment l'un de l'autre un atome d'hydrogène ou un radical alkyl linéaire ou ramifié en C₁-C₅,
   R₂ désigne un radical alkyle linéaire ou ramifié en C₁-C₄,
   R₃, R₄ et R₅ désignent indépendamment l'un de l'autre un atome d'hydrogène, un radical alkyle linéaire ou ramifié en C₁-C₃₀ ou un radical de formule (III) : 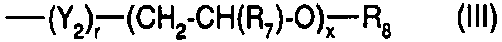
   Y, Y₁ et Y₂ désignent indépendamment l'un de l'autre un radical alkylène linéaire ou ramifié en C₂-C₁₆.
   R₇ désigne hydrogène, un radical alkyle linéaire ou ramifié en C₁-C₄ ou un radical hydroxyalkyle linéaire ou ramifié en C₁-C₄.
   R₈ désigne un atome d'hydrogène ou un radical alkyle linéaire ou ramifié en C₁-C₃₀,
   p, q et r désignent indépendamment l'un de l'autre soit la valeur 0 soit la valeur 1,
   m et n désignent indépendamment l'un de l'autre un nombre entier allant de 0 à 100,
   x désigne un nombre entier allant de 1 à 100,
   Z désigne un anion d'un acide organique ou minéral,
   sous réserve que :
   - un au moins des substituants R₃, R₄, R₅ ou R₈ désigne un radical alkyle linéaire ou ramifié en C₉ -C₃₀,
   - si m ou n est différent de 0 alors q est égal à 1,
   - si m ou n sont égaux à 0 alors l'un de p ou q est égal à 0.

Les différents objets de l'invention vont maintenant être détaillés. L'ensemble des significations et définitions des composés utilisés dans la présente invention données ci-dessous sont valables pour l'ensemble des objets de l'invention.

Sans vouloir être lié par une quelconque théorie, il semblerait que les avantages apportés par les polymères de l'invention soient en relation avec un comportement de polymères épaississants de type associatif. Les polymères associatifs sont des polymères dont les molécules sont capables, dans le milieu de formulation, de s'associer entre elles ou avec des molécules d'autres composés.

Leur structure chimique comprend généralement au moins une zone hydrophile et au moins une zone hydrophobe, la ou les zones hydrophobes comprenant au moins une chaîne grasse.

Les polymères poly(alkyl)vinyllactames cationiques selon l'invention comprennent :
1.-a) au moins un monomère de type vinyl lactame ou alkyl(C₁-C₅)vinyllactame,
2.-b) au moins un monomère choisi parmi ceux de structure (I) et (II) 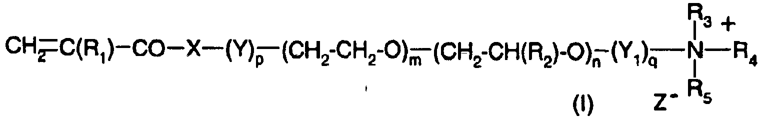 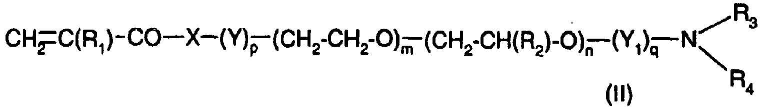 dans lesquelles :
   X désigne un atome d'oxygène ou un radical NR₆,
   R₁ et R₆ désignent indépendamment l'un de l'autre un atome d'hydrogène ou un radical alkyl linéaire ou ramifié en C₁-C₅,
   R₂ désigne un radical alkyle linéaire ou ramifié en C₁-C₄,
   R₃, R₄ et R₅ désignent indépendamment l'un de l'autre un atome d'hydrogène, un radical alkyle linéaire ou ramifié en C₁-C₃₀ ou un radical de formule (III) : 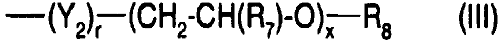
   Y, Y₁ et Y₂ désignent indépendamment l'un de l'autre un radical alkylène linéaire ou ramifié en C₂-C₁₆.
   R₇ désigne hydrogène, un radical alkyle linéaire ou ramifié en C₁-C₄ ou un radical linéaire ou ramifié hydroxyalkyle en C₁-C₄.
   R₈ désigne hydrogène ou un radical alkyle linéaire ou ramifié en C₁-C₃₀,
   p, q et r désignent indépendamment l'un de l'autre soit la valeur 0 soit la valeur 1,
   m et n désignent indépendamment l'un de l'autre un nombre entier allant de 0 à 100,
   x désigne un nombre entier allant de 1 à 100,
   Z désigne un anion d'un acide organique ou minéral,
   sous réserve que :
   ∘ un au moins des substituants R₃, R₄, R₅ ou R₈ désigne un radical alkyle linéaire ou ramifié en Cg -C₃₀,
   ∘ si m ou n est différent de 0 alors q est égal à 1,
   ∘ si m ou n sont égaux à 0 alors l'un de p ou q est égal à 0.

Les polymères selon l'invention peuvent être réticulés ou non et peuvent aussi être des polymères blocs.

De préférence le contre ion Z- des monomères de formule (I) est choisi parmi les ions halogénures, les ions phosphates, l'ion méthosulfate, l'ion tosylate.

De préférence R₃, R₄ et R₅ dans les formules (I) et/ou (II) désignent indépendamment l'un de l'autre un atome d'hydrogène ou un radical alkyle linéaire ou ramifié en C₁-C₃₀. Plus préférentiellement le monomère b) est un monomère de formule (I) et encore plus préférentiellement m et n sont égaux à 0.

Le monomère vinyl lactame ou alkylvinyllactame est de préférence un composé de structure (IV) : 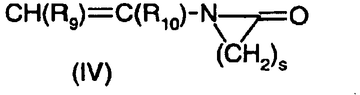 dans laquelle :
s désigne un nombre entier allant de 3 à 6
R₉ désigne un atome d'hydrogène ou un radical alkyle en C₁-C₅,
R₁₀ désigne un atome d'hydrogène ou un radical alkyle en C₁-C₅,
Sous réserve que l'un au moins des radicaux R₉ et R₁₀ désigne un atome d'hydrogène.

Encore plus préférentiellement le monomère (IV) est la vinylpyrrolidone.

Les polymères selon l'invention peuvent également contenir un ou plusieurs monomères supplémentaires de préférence cationiques ou non ioniques.

A titre de composés plus particulièrement préférés on citera les terpolymères comprenant au moins :
a)-un monomère de formule (IV).
b)-un monomère de formule (I) dans laquelle p=1, q=0, R₃ et R₄ désignent un atome d'hydrogène ou un radical alkyle en C₁-C₅ et R₅ désigne un radical alkyle en C₉-C₂₄, m et n sont nuls et
c)-un monomère de formule (II) dans laquelle R₃ et R₄ désignent un atome d'hydrogène ou un radical alkyle en C₁-C₅, m et n sont nuls.

Encore plus préférentiellement on utilisera les terpolymères poly(alkyl)vinyllactames comprenant en poids 40 à 95% de monomère (a), 25 à 50% de monomère (b) et 0,1 à 55% de monomère (c). De tels polymères sont décrits dans la demande de brevet WO 00/68282 dont le contenu fait partie intégrante de l'invention.

Comme polymères poly(alkyl)vinyllactames cationiques selon l'invention on utilisera notamment les terpolymères vinylpyrrolidone / diméthylaminopropyl méthacrylamide / tosylate de dodécyldiméthylméthacrylamidopropylammonium, les terpolymères vinylpyrrolidone / diméthylaminopropyl méthacrylamide / tosylate de cocoyl diméthyl méthacrylamidopropylammonium, les terpolymères vinylpyrrolidone / diméthylaminopropyl méthacrylamide / tosylate ou chlorure de lauryl diméthyl méthacrylamidopropylammonium.

La masse moléculaire en poids des polymères cationiques de l'invention est de préférence comprise entre 500 et 20 000 000. Elle est plus particulièrement compris entre 200 000 et 2 000 000 et encore plus préférentiellement compris entre 400 000 et 800 000.

Les polymères poly(alkyl)vinyllactames cationiques sont utilisés de préférence en une quantité pouvant varier d'environ 0,01 à 10% en poids du poids total de la composition de traitement des matières kératiniques. Plus préférentiellement, cette quantité varie d'environ 0,1 à 5% en poids.

Dans le cadre de la présente demande, on entend par agent conditionneur tout agent ayant pour fonction l'amélioration des propriétés cosmétiques des cheveux, en particulier la douceur, le démêlage, le toucher, l'électricité statique.

Les agents de conditionnement peuvent se présenter sous forme liquide, semi-solide ou solide tels que par exemple des huiles, des cires ou des gommes.

Selon l'invention, les agents conditionneurs sont choisis parmi les tensioactifs cationiques et les polysiloxanes à groupements aminés.

Parmi les tensioactifs cationiques, on peut citer en particulier : les sels d'amines grasses primaires, secondaires ou tertiaires, éventuellement polyoxyalkylénées ; les sels d'ammonium quaternaire ; les dérivés d'imidazoline ; ou les oxydes d'amines à caractère cationique.

Les sels d'ammonium quaternaires sont par exemple,:
- la formule générale (XVIII) suivante : 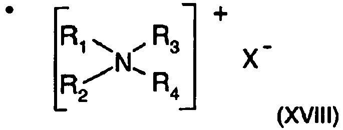 dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alkyl(C₁₂-C₂₂)acétate, hydroxyalkyle, comportant environ de 1 à 30 atomes de carbone; X est un anion choisi dans le groupe des halogénures, phosphates, acétates, lactates, alkyl(C₂-C₆)sulfates, alkyl-ou-alkylarylsulfonates,
   ∘ les sels d'ammonium quaternaire de l'imidazolinium, comme par exemple celui de formule (XIX) suivante : 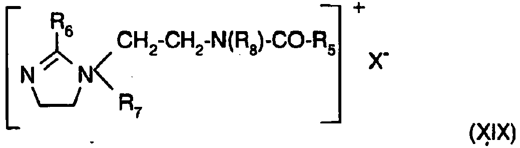 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple commercialisé sous la dénomination «REWOQUAT W 75» par la société REWO,
   ∘ les sels de diammonium quaternaire de formule (XX) : 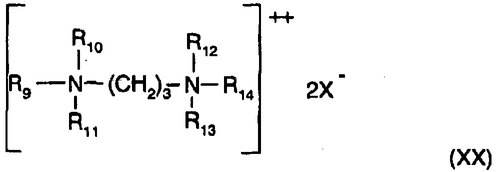 dans laquelle R₉ désigne un radical, aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
   ∘ les sels d'ammonium quaternaire contenant au moins une fonction ester

Les sels d'ammonium quaternaire contenant au moins une fonction ester utilisables selon l'invention sont par exemple ceux de formule (XXI) suivante : 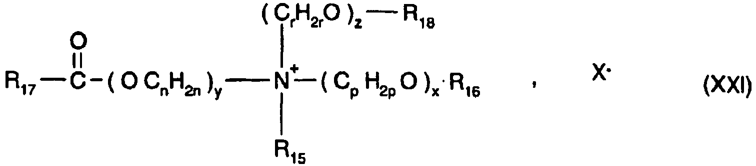 dans laquelle :
- R₁₅ est choisi parmi les radicaux alkyles en C₁-C₆ et les radicaux hydroxyalkyles ou dihydroxyalkyles en C₁-C₆ ;
- R₁₆ est choisi parmi :
   ∘ le radical 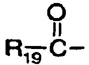
   ∘ les radicaux R₂₀ hydrocarbonés en C₁-C₂₂ linéaires ou ramifiés, saturés ou insaturés,
   ∘ l'atome d'hydrogène,
- R₁₈ est choisi parmi :
   ∘ le radical 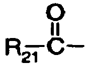
   ∘ les radicaux R₂₂ hydrocarbonés en C₁-C₆ linéaires ou ramifiés, saturés ou insaturés,
   ∘ l'atome d'hydrogène,
- R₁₇, R₁₉ et R₂₁, identiques ou différents, sont choisis parmi les radicaux hydrocarbonés en C₇-C₂₁, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X⁻ est un anion simple ou complexe, organique ou inorganique ;
sous réserve que la somme x + y + z vaut de 1 à 15 , que lorsque x vaut 0 alors R₁₆ désigne R₂₀ et que lorsque z vaut 0 alors R₁₈ désigne R₂₂.

Les radicaux alkyles R₁₅ peuvent. être linéaires ou ramifiés et plus particulièrement linéaires.

De préférence R₁₅ désigne un radical méthyle, éthyle, hydroxyéthyle ou dihydroxypropyle et plus particulièrement un radical méthyle ou éthyle.

Avantageusement, la somme x + y + z vaut de 1 à 10.

Lorsque R₁₆ est un radical R₂₀ hydrocarboné, il peut être long et avoir de 12 à 22 atomes de carbone ou court et avoir de 1 à 3 atomes de carbone.

Lorsque R₁₈ est un radical R₂₂ hydrocarboné, il a de préférence 1 à 3 atomes de carbone.

Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1. Avantageusement, y est égal à 1. De préférence, n, p et r, identiques ou différents, valent 2 ou 3 et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure du le méthylsulfate.

On utilise plus particulièrement les sels d'ammonium de formule (XXI) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   ∘ le radical 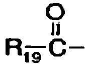
   ∘ les radicaux méthyle, éthyle ou hydrocarbonés en C₁₄-C₂₂
   ∘ l'atome d'hydrogène ;
- R₁₈ est choisi parmi :
   ∘ le radical 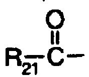
   ∘ l'atome d'hydrogène ;

R₁₇, R₁₉ et R₂₁, identiques ou différents, sont choisis parmi les radicaux hydrocarbonés en C₁₃-C₁₇, linéaires ou ramifiés, saturés ou insaturés et de préférence parmi les radicaux alkyles et alcényle en C₁₃-C₁₇, linéaires ou ramifiés, saturés ou insaturés.
Avantageusement, les radicaux hydrocarbonés sont linéaires.

On peut citer par exemple les composés de formule (XXI) tels que les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus par exemple par estérification directe de la triéthanolamine, de la trisopropanolamine, d'alkyldiéthanolamine ou d'alkyldiisopropanolamine éventuellement oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART par la société HENKEL, STEPANQUAT par la société STEPAN, NOXAMIUM par la société CECA, REWOQUAT WE 18 par la société REWO-WITCO.

On peut aussi utiliser les sels d'ammonium contenant au moins une fonction ester décrits dans les brevets US-A-4874554 et US-A-4137180.

Parmi les sels d'ammonium quaternaire de formule (XVIII) on préfère, d'une part, les chlorures de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore, d'autre part, le chlorure de stéaramidopropyldiméthyl (myristyl acétate) ammonium commercialisé sous la dénomination «CERAPHYL 70» par la société VAN DYK.

Il est bien entendu possible de mettre en oeuvre des mélanges d'agents conditionneur.

Selon l'invention, le ou les agents conditionneurs peuvent représenter de 0,001 % à 20 % en poids, de préférence de 0,01 % à 10% en poids et plus particulièrement de 0,1 à 3% en poids par rapport au poids total de la composition finale.

Les compositions de l'invention contiennent en outre avantageusement au moins un agent tensioactif qui est généralement présent en une quantité comprise entre 0,1% et 60% en poids environ, de préférence entre 1% et 40% et encore plus préférentiellement entre 5% et 30%, par rapport au poids total de la composition.

Cet agent tensioactif peut être choisi parmi les agents tensioactifs anioniques, amphotères, non-ioniques, ou leurs mélanges.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique. Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures:

R₂-CONHCH₂CH₂-N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou uhdécyle, R₃ désigne un groupement bêta-hydroxyéthyle et R₄ un groupement carboxyméthyle ; et

R₅-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO3H
R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid. A titre d'exemple on peut citer le cocoamphodiacetate commercialisé sous la dénomination commerciale MIRANOL C2M concentré par la société RHONE POULENC.

Dans les compositions conformes à l'invention, on utilise de préférence des mélanges d'agents tensioactifs et en particulier des mélanges d'agents tensioactifs anioniques et des mélanges d'agents tensioactifs anioniques et d'agents tensioactifs amphotères ou non ioniques. Un mélange particulièrement préféré est un mélange constitué d'au moins un argent tensioactif anionique et d'au moins un agent tensioactif amphotère.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHONE POULENC sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société HENKEL.

La composition de l'invention peut également contenir au moins un additif choisi parmi les épaississants, les parfums, les agents nacrants, les conservateurs, les polymères anioniques ou non ioniques, les protéines non cationiques, les hydrolysats de protéines non cationiques, l'acide méthyl-18 eicosanoique, les hydroxyacides, d'autres polymères que ceux de l'invention et en particulier des polyuréthanes associatifs cationiques ou non ioniques polyéther et tout autre additif classiquement utilisé dans le domaine cosmétique qui n'affecte pas les propriétés des compositions selon l'invention.

Ces additifs sont présents dans la composition selon l'invention dans des proportions pouvant aller de 0 à 20% en poids par rapport au poids total de la composition. La quantité précise de chaque additif est déterminée facilement par l'homme du métier selon sa nature et sa fonction.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

En particulier, les compositions selon l'invention sont des compositions détergentes telles que des shampooings, des gels-douche et des bains moussants. Dans ce mode de réalisation de l'invention, les compositions comprennent une base lavante, généralement aqueuse.

Le ou les tensioactifs formant la base lavante peuvent être indifféremment choisis, seuls ou en mélanges, au sein des tensioactifs anioniques, amphotères, non ioniques tels que définis ci-dessus.

La quantité et la qualité de la base lavante sont celles suffisantes pour conférer à la composition finale un pouvoir moussant et/ou détergent satisfaisant.

Ainsi, selon l'invention, la base lavante peut représenter de 4 % à 50 % en poids, de préférence de 6 % à 35 % en poids, et encore plus préférentiellement de 8 % à 25 % en poids, du poids total de la composition finale

Le pH de la composition appliquée sur les matières kératiniques est généralement compris entre les valeurs 2 et 11. Il est de préférence compris entre 3 et 8, et peut être ajusté à la valeur désirée au moyen d'agents acidifiants ou alcalinisants bien connus de l'état de la technique des compositions appliquées sur des matières kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule (XXII) suivante : 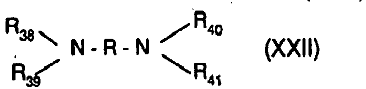 dans laquelle R est un reste propylène éventuellement substitué par un groupement hydroxyle ou un radical alkyle en C₁-C₄ ; R₃₈, R₃₉, R₄₀ et R₄₁, identiques ou différents, représentent un atome d'hydrogène, un radical alkyle en C₁-C₄ ou hydroxyalkyle en C₁-C_{4.}

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le milieu physiologiquement et en particulier cosmétiquement acceptable peut être constitué uniquement par de l'eau, par un solvant cosmétiquement acceptable ou par un mélange d'eau et d'un solvant cosmétiquement acceptable tel que notamment un alcool inférieur en C₁-C₄, comme l'éthanol, l'isopropanol, le tertiobutanol, le n-butanol ; les alkylèneglycols comme le propylèneglycol, les éthers de glycols.

L'invention a aussi pour objet un procédé de traitement des matières kératiniques telles que la peau ou les cheveux, **caractérisé en ce qu**'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage ou le démaquillage de la peau, des cheveux ou de toute autre matière kératinique.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Dans tout ce qui suit ou ce qui précède, les pourcentages exprimés sont en poids. L'invention va être maintenant plus complètement illustrée à l'aide des exemples suivants qui ne sauraient être considérés comme la limitant aux modes de réalisation décrits. Dans les exemples, MA signifie matière active.

Le polymère 1 est un terpolymère vinylpyrrolidone / diméthyl amino propylméthacrylamide/ chlorure de lauryldiméthylméthacrylamidoammonium proposé par la Société ISP sous la référence POLYMER ACP-1234.

### EXEMPLE 1 (ne fait pas parti de l'invention)

On réalise une composition de shampooing :

| | |
|---|---|
| - Lauryléthersulfate de sodium à 2,2 moles d'oxyde d'éthylène à 28% de MA | 17 gMA |
| - Cocoylbétaïne à 30% de MA | 2,5 gMA |
| - Polymère 1 | 1 g MA |
| - Monoisopropanolamide d'acides de coprah | 0,6 g |
| - Acide 2-hydroxy 4-méthoxybenzophénone-5-sulfonique (UVINUL MS 40 de BASF) | 0,1 g |
| - Parfum, conservateurs | qs |
| - Eau déminéralisée qsp | 100 g |

Les cheveux traités avec ce shampooing sont lisses, doux et protégés de l'action néfaste de la lumière.

### EXEMPLE 2 (ne fait pas parti de l'invention)

On réalise la composition de shampooing suivante :

| | |
|---|---|
| - Lauryléthersulfate de sodium à 2,2 moles d'oxyde d'éthylène à 30% MA | 10 gMA |
| - Cocoylbétaïne à 30%MA | 4 gMA |
| - Polymère 1 | 0,5 g MA |
| - Polydiméthylsiloxane de viscosité 300.000 cSt (Silicone AK300000 de WACKER) | 0,5 g |
| - Gomme de xanthane | 1 g |
| - Acide citrique qs pH | 7 |
| - Eau déminéralisée qsp | 100 g |

Les cheveux traités avec ce shampooing sont lisses et doux.

### EXEMPLE 3

On réalise un après-shampooing conforme à l'invention de composition suivante :
- Polymère 1 0,5 g MA
- Chlorure de béhényl triméthyl ammonium 1,5 g MA
- Mélange d'alcool cétyl stéarylique et d'alcool cétyl stéarylique oxyéthyléné 33 OE (80/20) 4 g
- Eau déminéralisée qsp 100 g

Les cheveux traités avec cet après-shampooing sont lisses et doux.

### EXEMPLE 4

On réalise un après-shampooing conforme à l'invention de composition suivante :
- Polymère 1 0,5 gMA
- Chlorure de béhényl triméthyl ammonium 1,5 g MA
- N-oléoyl dihydrosphingosine 0,5 g
- Eau qsp 100 g

Les cheveux traités avec cet après-shampooing sont lisses et doux.

## Revendications

1. Composition de traitement des matières kératiniques, en particulier des fibres kératiniques humaines telles que les cheveux, comprenant dans un milieu physiologiquement et en particulier cosmétiquement acceptable au moins un agent conditionneur choisi parmi les tensioactits cationiques et les polysiloxanes à groupements aminés et au moins un polymère poly(alkyl)vinyllactame cationique comprenant :
- a) au moins un monomère de type vinyl lactame ou alkyl(C₁-C₅)vinyllactame,
- b) au moins un monomère choisi parmi ceux de structures (I) et (II) : 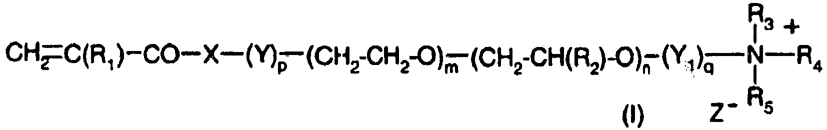 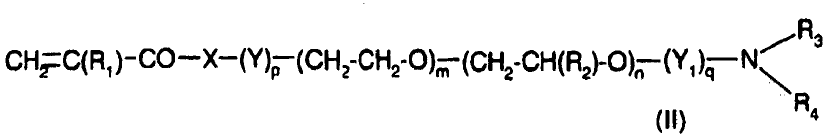 dans lesquelles :
X désigne un atome d'oxygène ou un radical NR₆,
R₁ et R₆ désignent indépendamment l'un de l'autre un atome d'hydrogène ou un radical alkyl linéaire ou ramifié en C₁-C₅,
R₂ désigne un radical alkyle linéaire ou ramifié en C₁-C₄,
R₃, R₄ et R₅ désignent indépendamment l'un de l'autre un atome d'hydrogène, un radical alkyle linéaire ou ramifié en C₁-C₃₀ ou un radical de formule (III) : 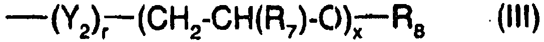
Y, Y₁ et Y₂ désignent indépendamment l'un de l'autre un radical alkylène linéaire ou ramifié en C₂-C_{16.}
R₇ désigne hydrogène, un radical alkyle linéaire ou ramifié en C₁-C₄ ou un radical hydroxyalkyle linéaire ou ramifié en C₁-C₄.
R₈ désigne hydrogène ou un radical alkyle linéaire ou ramifié en C₁-C₃₀.
p, q et r désignent indépendamment l'un de l'autre soit la valeur 0 soit la valeur 1,
m et n désignent indépendamment l'un de l'autre un nombre entier allant de 0 à 100,
x désigne un nombre entier allant de 1 à 100,
Z désigne un anion d'un acide organique ou minéral,
sous réserve que ;
- un au moins des substituants R₃, R₄, R₅ ou R₈ désigne un radical alkyle linéaire ou ramifié en C₉ -C₃₀,
- si m ou n est différent de 0 alors q est égal à 1,
- si m ou n sont égaux à 0 alors l'un de p ou q est égal à 0.

2. Composition selon la revendication 1 **caractérisée par le fait que le** monomère vinyl lactame ou alkylvinyllactame est un composé de structure (IV) : 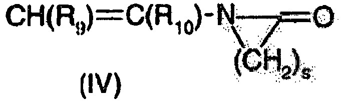 dans laquelle :
s désigne un nombre entier allant de 3 à 6
R₉ désigne un atome d'hydrogène ou un radical alkyle en C₁-C₅,
R₁₀ désigne un atome d'hydrogène ou un radical alkyle en C₁-C₅,
Sous réserve que l'un au moins des radicaux R₉ et R₁₀ désigne un atome d'hydrogène.

3. Composition selon la revendication 2 **caractérisée par le fait que** le monomère de formule (IV) est la vinylpyrrolidone.

4. Composition selon l'une quelconque des revendications 1 à 3, **caractérisée par le fait que** R₃, R₄ et R₅ dans les formules (I) et/ou (II) désignent indépendamment l'un de l'autre un atome d'hydrogène ou un radical alkyle linéaire ou ramifié en C₁-C₃₀.

5. Composition selon l'une quelconque des revendications 1 à 4, **caractérisée par le fait que le** monomère b) est un monomère de formule (I).

6. Composition selon l'une quelconque des revendications 1 à 5, **caractérisée par le fait que** les nombres m et n sont égaux à 0.

7. Composition selon l'une quelconque des revendications 1 à 6, **caractérisée par le fait que** le contre ion Z- des monomères de formule (I) est choisi parmi les ions halogénures, les ions phosphates, l'ion méthosulfate, l'iontosylate

8. Composition selon l'une quelconque des revendications 1 à 7, **caractérisée par le fait que** le ou les polymère cationiques selon l'invention contiennent également un ou plusieurs monomères supplémentaires de préférence cationiques ou non ioniques

9. Composition selon l'une quelconque des revendications 1 à 8, **caractérisée par le fait que** le polymère est un terpolymère comprenant :
a)-un monomère de formule (IV).
b)-un monomère de formule (I) dans laquelle p=1, q=0, R₃ et R₄ désignent un atome d'hydrogène ou un radical alkyle en C₁-C₅ et R₅ désigne un radical alkyle en C₉-C₂₄ et
c)-un monomère de formule (II) dans laquelle R₃ et R₄ désigne un atome d'hydrogène ou un radical alkyle en C₁-C₅.

10. Composition selon la revendication 9, **caractérisée par le fait que** le terpolymère comprend en poids 40 à 95% de monomère (a), 0,25 à 50% de monomère (b) et 0,1 à 55% de monomère (c).

11. Composition selon l'une quelconque des revendications 1 à 10, **caractérisée par le fait que** les polymères poly (alkyl)vinyllactames cationiques sont les terpolymères vinylpyrrolidone / diméthylaminopropylméthacrylamide / tosylate de dodécyldiméthylméthacrylamidopropylammonium, les terpolymères vinylpyrrolidone / diméthylaminopropyl méthacrylamide/tosylate de cocoyl diméthyl méthacrylamidopropylammonium, les terpolymères vinylpyrrolidone / diméthylaminopropyl méthacrylamide / tosylate ou chlorure de lauryl diméthyl méthacrylamidopropylammonium.

12. Composition selon l'une quelconque des revendications 1 à 11, **caractérisée par le fait que** la masse moléculaire en poids des polymères cationiques est comprise entre 500 et 20 000 000, de préférence comprise entre 200 000 et 2 000 000 et encore plus préférentiellement comprise entre 400 000 et 800 000.

13. Composition selon l'une quelconque des revendications précédentes, **caractérisée par le fait que** les polymères poly(alkyl)vinyllactames cationiques sont utilisés en une quantité variant de 0,01 à 10% en poids du poids total de la composition.

14. Composition selon la revendication 13, **caractérisée par le fait que** les polymères poly(alkyl)vinyllactames cationiques sont utilisés en une quantité variant de 0,1 à 5% en poids du poids total de la composition.

15. Composition selon l'une quelconque des revendications précédentes, **caractérisée en ce que** le ou les agents conditionneurs sont présents à une concentration comprise entre 0,001 % et 20 % en poids par rapport au poids total de la composition, de préférence entre 0,01 % et 10 % en poids.

16. Composition selon l'une quelconque des revendications précédentes, **caractérisée par le fait qu'**elle comprend en outre au moins un agent tensioactif choisi parmi les tensioactifs anioniques, non ioniques, amphotères et leurs mélanges.

17. Composition selon la revendication 16, **caractérisée par le fait que** le ou les agents tensioactifs sont présents à une concentration comprise entre 0,1% et 60% en poids, de préférence entre 1% et 40% en poids, et encore plus préférentiellement entre 5% et 30% en poids, par rapport au poids total de la composition.

18. Composition selon l'une quelconque des revendications précédentes, **caractérisée par le fait qu'elle** se présente sous forme de shampooing, d'après-shampooing, de composition pour la permanente, le défrisage, la coloration ou la décoloration des cheveux, de composition à rincer à appliquer entre les deux étapes d'une permanente ou d'un défrisage, de composition lavantes pour le corps.

19. Utilisation d'une composition telle que définie dans l'une quelconque des revendications précédentes pour le lavage ou pour le soin des matières kératiniques.

20. Procédé de traitement des matières kératiniques, telles que les cheveux, **caractérisé en ce qu'il** consiste à appliquer sur lesdites matières une composition cosmétique selon l'une des revendications 1 à 18, puis à effectuer éventuellement un rinçage à l'eau.

## Patentansprüche

1. Zusammensetzung zur Behandlung von Keratinsubstanzen und insbesondere menschlichen Keratinfasern wie Haaren, die in einem physiologisch und insbesondere kosmetisch akzeptablen Medium mindestens ein Konditioniermittel, das unter den kationischen grenzflächenaktiven Stoffen und den Polysiloxanen mit aminierten Gruppen ausgewählt ist, und mindestens ein kationisches Poly(alkyl)vinyllactampolymer enthält, das umfasst:
- a) mindestens ein Monomer vom Vinyllactamtyp oder Alkyl (C₁-₅) -vinyllactamtyp,
- b) mindestens ein Monomer, das unter den Monomeren der folgenden Strukturen (I) und (II) ausgewählt ist: 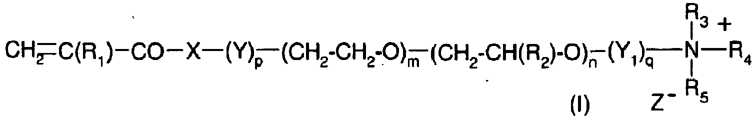 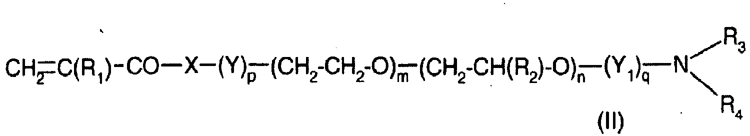 worin bedeuten:
X ein Sauerstoffatom oder eine Gruppe NR₆,
R₁ und R₆ unabhängig voneinander ein Wasserstoffatom oder eine geradkettige oder verzweigte C₁-₅-Alkylgruppe,
R₂ eine geradkettige oder verzweigte C₁₋₄-Alkylgruppe, R₃, R₄ und R₅ unabhängig voneinander ein Wasserstoffatom, eine geradkettige oder verzweigte C₁₋₃₀-Alkylgruppe oder eine Gruppe der Formel (III) : 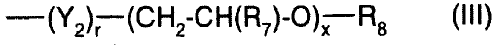
Y, Y₁ und Y₂ unabhängig voneinander eine geradkettige oder verzweigte C₂₋₁₆-Alkylengruppe,
R₇ Wasserstoff, eine geradkettige oder verzweigte C₁₋₄-Alkylgruppe oder eine geradkettige oder verzweigte C₁₋₄-Hydroxyalkylgruppe,
R₈ Wasserstoff oder eine geradkettige oder verzweigte C₁₋₃₀-Alkylgruppe,
p, q und r unabhängig voneinander entweder Null oder 1,
m und n unabhängig voneinander eine ganze Zahl von 0 bis 100,
x eine ganze Zahl von 1 bis 100,
Z ein Anion einer organischen oder anorganischen Säure, mit der Maßgabe, dass:
- mindestens ein Substituent R₃, R₄, R₅ oder R₈ eine geradkettige oder verzweigte C₉₋₃₀-Alkylgruppe bedeutet,
- q 1 bedeutet, wenn m oder n von Null verschieden ist,
- eines von p oder q Null bedeutet, wenn m oder n 0 ist.

2. Zusammensetzung nach Anspruch 1, **dadurch gekennzeichnet, dass** das Vinyllactammonomer oder Alkylvinyllactammonomer eine Verbindung der Struktur (IV) ist: 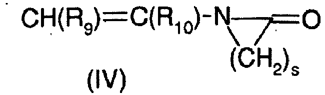 worin bedeuten:
s eine ganze Zahl von 3 bis 6,
R₉ ein Wasserstoffatom oder eine C₁₋₅-Alkylgruppe,
R₁₀ ein Wasserstoffatom oder eine C₁₋₅-Alkylgruppe, mit der Maßgabe, dass mindestens eine Gruppe R₉ oder R₁₀ ein Wasserstoffatom bedeutet.

3. Zusammensetzung nach Anspruch 2, **dadurch gekennzeichnet, dass** das Monomer der Formel (IV) das Vinylpyrrolidon ist.

4. Zusammensetzung nach einem der Ansprüche 1 bis 3, **dadurch gekennzeichnet, dass** in den Formeln (I) und/oder (II) R₃, R₄ und R₅ unabhängig voneinander ein Wasserstoffatom oder eine geradkettige oder verzweigte C₁₋₃₀-Alkylgruppe bedeuten.

5. Zusammensetzung nach einem der Ansprüche 1 bis 4, **dadurch gekennzeichnet, dass** das Monomer b) ein Monomer der Formel (I) ist.

6. Zusammensetzung nach einem der Ansprüche 1 bis 5, **dadurch gekennzeichnet, dass** m und n Null bedeuten.

7. Zusammensetzung nach einem der Ansprüche 1 bis 6, **dadurch gekennzeichnet, dass** das Gegenion Z⁻ der Monomere der Formel (I) unter den Halogenidionen, Phosphationen, dem Methosulfation oder dem Tosylation ausgewählt ist.

8. Zusammensetzung nach einem der Ansprüche 1 bis 7, **dadurch gekennzeichnet, dass** das oder die erfindungsgemäße(n) kationische(n) Polymer(e) ferner ein oder mehrere zusätzliche Monomere, insbesondere ein oder mehrere zusätzliche kationische oder nichtionische Monomere, enthalten.

9. Zusammensetzung nach einem der Ansprüche 1 bis 8, **dadurch gekennzeichnet, dass** das Polymer ein Terpolymer ist, das enthält:
a) ein Monomer der Formel (IV),
b) ein Monomer der Formel (I) mit p=1, q=0 und worin R₃ und R₄ ein Wasserstoffatom oder eine C₁₋₅-Alkylgruppe bedeuten und R₅ eine C₉₋₂₄-Alkylgruppe bedeutet, und
c) ein Monomer der Formel (II), worin R₃ und R₄ ein Wasserstoffatom oder eine C₁₋₅-Alkylgruppe bedeuten.

10. Zusammensetzung nach Anspruch 9, **dadurch gekennzeichnet, dass** das Terpolymer 40 bis 95 Gew.-% Monomer (a), 0,25 bis 50 Gew.-% Monomer (b) und 0,1 bis 55 Gew.-% Monomer (c) enthält.

11. Zusammensetzung nach einem der Ansprüche 1 bis 10, **dadurch gekennzeichnet, dass** es sich bei den kationischen Poly(alkyl)vinyllactampolymeren um die Terpolymere Vinylpyrrolidon/Dimethylaminopropylmethacrylamid/Dodecyldimethylmethacrylamidopropylammoniumtosylat, die Terpolymere Vinylpyrrolidon/Dimethylaminopropylmethacrylamid/Cocoyldimethylmethacrylamidopropylammoniumtosylat, die Terpolymere Vinylpyrrolidon/ Dimethylaminopropylmethacrylamid/Lauryldimethylmethacrylamidopropylammoniumtosylat oder Lauryldimethylmethacrylamidopropylammoniumchlorid handelt.

12. Zusammensetzung nach einem der Ansprüche 1 bis 11, **dadurch gekennzeichnet, dass** das Molekulargewicht der kationischen Polymere im Bereich von 500 bis 20 000 000, vorzugsweise im Bereich von 200 000 bis 2 000 000 und noch bevorzugter im Bereich von 400 000 bis 800 000 liegt.

13. Zusammensetzung nach einem der vorhergehenden Ansprüche, **dadurch gekennzeichnet, dass** die kationischen Poly(alkyl)vinyllactampolymere in einer Menge von 0,01 bis 10 Gew.-% des Gesamtgewichts der Zusammensetzung verwendet werden.

14. Zusammensetzung nach Anspruch 13, **dadurch gekennzeichnet, dass** die kationischen Poly(alkyl)vinyllactame in einer Menge von 0,1 bis 5 Gew.-% des Gesamtgewichts der Zusammensetzung verwendet werden.

15. Zusammensetzung nach einem der vorhergehenden Ansprüche, **dadurch gekennzeichnet, dass** das oder die Konditioniermittel in einer Konzentration von 0,001 bis 20 Gew.-%, bezogen auf das Gesamtgewicht der Zusammensetzung, und vorzugsweise 0,01 bis 10 Gew.-% vorliegen.

16. Zusammensetzung nach einem der vorhergehenden Ansprüche, **dadurch gekennzeichnet, dass** sie ferner mindestens einen grenzflächenaktiven Stoff enthält, der unter den anionischen, nichtionischen oder amphoteren grenzflächenaktiven Stoffen und deren Gemischen ausgewählt ist.

17. Zusammensetzung nach Anspruch 16, **dadurch gekennzeichnet, dass** der oder die grenzflächenaktiven Stoffe in einer Konzentration im Bereich von 0,1 bis 60 Gew.-%, vorzugsweise im Bereich von 1 bis 40 Gew.-% und noch bevorzugter im Bereich von 5 bis 30 Gew.-%, bezogen auf das Gesamtgewicht der Zusammensetzung, vorliegen.

18. Zusammensetzung nach einem der vorhergehenden Ansprüche, **dadurch gekennzeichnet, dass** sie als Haarwaschmittel, Conditioner, Zusammensetzung für dauerhafte Verformung, Entkräuselung, Färbung oder Entfärbung der Haare, Zusammensetzung, die ausgespült wird und zwischen den beiden Schritten einer permanenten Verformung oder Entkräuselung aufgebracht wird, oder reinigende Zusammensetzung für den Körper vorliegt.

19. Verwendung einer Zusammensetzung nach einem der vorhergehenden Ansprüche für die Reinigung oder die Pflege von Keratinsubstanzen.

20. Verfahren zur Behandlung von Keratinsubstanzen, wie Haaren, **dadurch gekennzeichnet, dass** es darin besteht, eine kosmetische Zusammensetzung nach einem der Ansprüche 1 bis 18 auf die Substanzen aufzutragen und gegebenenfalls anschließend mit Wasser zu spülen.

## Claims

1. Composition for treating keratin materials, in particular human keratin fibers such as the hair, comprising, in a physiologically and in particular a cosmetically acceptable medium, at least one conditioning agent chosen from cationic surfactants and polysiloxanes containing amine groups and at least one cationic poly(alkyl)vinyllactam polymer comprising:
- a) at least one monomer of vinyllactam or (C₁-C₅)alkylvinyllactam type;
- b) at least one monomer chosen from those of structures (I) and (II): 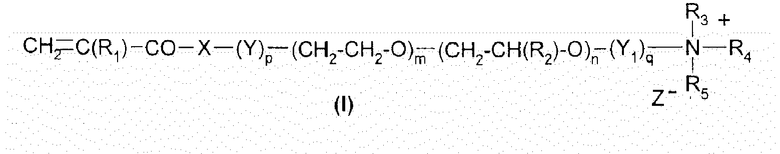 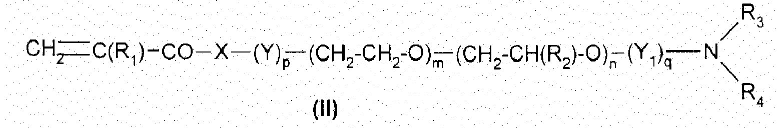 in which:
X denotes an oxygen atom or a radical NR₆,
R₁ and R₆ denote, independently of each other, a hydrogen atom or a linear or branched C₁-C₅ alkyl radical,
R₂ denotes a linear or branched C₁-C₄ alkyl radical, R₃, R₄ and R₅ denote, independently of each other, a hydrogen atom, a linear or branched C₁-C₃₀ alkyl radical or a radical of formula (III):
-(Y₂)ᵣ-(CH₂-CH(R₇)-O)ₓ-R₈ (III)
Y, Y₁ and Y₂ denote, independently of each other, a linear or branched C₂-C₁₆ alkylene radical,
R₇ denotes hydrogen, a linear or branched C₁-C₄ alkyl radical or a linear or branched C₁-C₄ hydroxyalkyl radical,
R₈ denotes a hydrogen atom or a linear or branched C₁-C₃₀ alkyl radical,
p, q and r denote, independently of each other, either the value 0 or the value 1,
m and n denote, independently of each other, an integer ranging from 0 to 100,
x denotes an integer ranging from 1 to 100,
Z denotes an organic or mineral acid anion,
with the proviso that:
- at least one of the substituents R₃, R₄, R₅ or R₈ denotes a linear or branched C₉-C₃₀ alkyl radical,
- if m or n is other than 0, then q is equal to 1,
- if m or n are equal to 0, then either p or q is equal to 0.

2. Composition according to Claim 1, **characterized in that** the vinyllactam or alkylvinyllactam monomer is a compound of structure (IV): 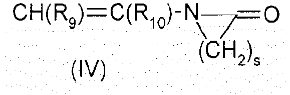 in which:
s denotes an integer ranging from 3 to 6,
R₉ denotes a hydrogen atom or a C₁-C₅ alkyl radical,
R₁₀ denotes a hydrogen atom or a C₁-C₅ alkyl radical,
with the proviso that at least one of the radicals R₉ and R₁₀ denotes a hydrogen atom.

3. Composition according to Claim 2, **characterized in that** the monomer of formula (IV) is vinylpyrrolidone.

4. Composition according to any one of Claims 1 to 3, **characterized in that** R₃, R₄ and R₅ in formulae (I) and/or (II) denote, independently of each other, a hydrogen atom or a linear or branched C₁-C₃₀ alkyl radical.

5. Composition according to any one of Claims 1 to 4, **characterized in that** the monomer b) is a monomer of formula (I).

6. Composition according to any one of Claims 1 to 5, **characterized in that** the numbers m and n are equal to 0.

7. Composition according to any one of Claims 1 to 6, **characterized in that** the counterion Z⁻ of the monomers of formula (I) is chosen from halide ions, phosphate ions, methosulfate ions and tosylate ions.

8. Composition according to any one of Claims 1 to 7, **characterized in that** the cationic polymer (s) according to the invention also contain (s) one or more additional monomer(s), which are preferably cationic or nonionic.

9. Composition according to any one of Claims 1 to 8, **characterized in that** the polymer is a terpolymer comprising:
a) one monomer of formula (IV),
b) one monomer of formula (I) in which p=1, q=0, R₃ and R₄ denote a hydrogen atom or a C₁-C₅ alkyl radical and R₅ denotes a C₉-C₂₄ alkyl radical, and
c) one monomer of formula (II) in which R₃ and R₄ denote a hydrogen atom or a C₁-C₅ alkyl radical.

10. Composition according to Claim 9, **characterized in that** the terpolymer comprises, by weight, 40% to 95% of monomer (a), 0.25% to 50% of monomer (b) and 0.1% to 55% of monomer (c).

11. Composition according to any one of Claims 1 to 10, **characterized in that** the cationic poly(alkyl)vinyllactam polymers are vinylpyrrolidone/dimethylaminopropylmethacrylamide/dodecyldimethylmethacrylamidopropylammonium tosylate terpolymers, vinylpyrrolidone/dimethylaminopropylmethacrylamide/cocoyldimethylmethacrylamidopropylammonium tosylate terpolymers, or vinylpyrrolidone/dimethylaminopropylmethacrylamide/lauryldimethylmethacrylamidopropylammonium tosylate or chloride terpolymers.

12. Composition according to any one of Claims 1 to 11, **characterized in that** the weight-average molecular mass of the cationic polymers is between 500 and 20 000 000, preferably between 200 000 and 2 000 000 and even more preferably between 400 000 and 800 000.

13. Composition according to any one of the preceding claims, **characterized in that** the cationic poly(alkyl)vinyllactam polymers are used in an amount ranging from 0.01% to 10% by weight relative to the total weight of the composition.

14. Composition according to Claim 13, **characterized in that** the cationic poly(alkyl)vinyllactam polymers are used in an amount ranging from 0.1% to 5% by weight relative to the total weight of the composition.

15. Composition according to any one of the preceding claims, **characterized in that** the conditioning agent(s) is (are) present in a concentration of between 0.001% and 20% by weight and preferably between 0.01% and 10% by weight relative to the total weight of the composition.

16. Composition according to any one of the preceding claims, **characterized in that** it also comprises at least one surfactant chosen from anionic, nonionic and amphoteric surfactants, and mixtures thereof.

17. Composition according to Claim 16, **characterized in that** the surfactant (s) is (are) present in a concentration of between 0.1% and 60% by weight, preferably between 1% and 40% by weight and even more preferably between 5% and 30% by weight, relative to the total weight of the composition.

18. Composition according to any one of the preceding claims, **characterized in that** it is in the form of a shampoo, a conditioner, a composition for permanent-waving, straightening, dyeing or bleaching the hair, a rinse-out composition to be applied between the two steps of a permanent-waving or hair-straightening operation, or a washing composition for the body.

19. Use of a composition as defined in any one of the preceding claims, for washing or caring for keratin materials.

20. Process for treating keratin materials, such as the hair, **characterized in that** it consists in applying to said materials a cosmetic composition according to one of Claims 1 to 18, optionally followed by rinsing with water.
